# EUROPÄISCHE PATENTSCHRIFT

(11) **EP 2 132 335 B1**
(45) Veröffentlichungstag und Bekanntmachung des Hinweises auf die Patenterteilung: **16.05.2012**
(21) Anmeldenummer: 08757923.1
(22) Anmeldetag: 29.03.2008
(51) Int. Cl.: C12Q 1/68

(54) **VERFAHREN ZUM NACHWEIS VON PARATUBERKULOSE**
METHOD FOR THE DETECTION OF PARATUBERCULOSIS
PROCÉDÉ DE DÉPISTAGE DE PARATUBERCULOSE

(30) Priorität: 30.03.2007 DE 102007015775
(43) Veröffentlichungstag der Anmeldung: 16.12.2009
(73) Patentinhaber: Justus-Liebig-Universität Gießen, 35390 Giessen (DE)
(72) Erfinder: BÜLTE, Michael, 35305 Reinhardshain (DE); ABDULMAWJOOD, Amir, 35396 Giessen (DE); SCHÖNENBRÜCHER, Holger, 63762 Grossostheim-Pflaumheim (DE)
(74) Vertreter: Buchhold, Jürgen
(86) Internationale Anmeldenummer: PCT/DE2008/000523
(87) Internationale Veröffentlichungsnummer: WO 2008/119332

(56) Entgegenhaltungen:
- WO-A-2006/108205
- SCHOENENBRUECHER H ET AL: "Molecular detection of mycobacterium avium ssp paratuberculosis: Development and validation of a Triplex-Real Time-PCR-assay" IJMM INTERNATIONAL JOURNAL OF MEDICAL MICROBIOLOGY, Bd. 296, Nr. Suppl. 42, September 2006 (2006-09), Seite 65, XP009107779 & 58TH ANNUAL CONFERENCE OF THE GERMAN-SOCIETY-OF-HYGIENE-AND-MICROBIO LOGY; WURZBURG, GERMANY; 200610, ISSN: 1438-4221
- FUELGRABE R ET AL: "Molecular and cultural detection of Mycobacterium avium ssp paratuberculosis (MAP) in patients with inflammatory bowel disease and in controls" IJMM INTERNATIONAL JOURNAL OF MEDICAL MICROBIOLOGY, Bd. 296, Nr. Suppl. 42, September 2006 (2006-09), Seite 90, XP009107778 & 58TH ANNUAL CONFERENCE OF THE GERMAN-SOCIETY-OF-HYGIENE-AND-MICROBIO LOGY; WURZBURG, GERMANY; 200610, ISSN: 1438-4221
- HERTHNEK DAVID ET AL: "New PCR systems to confirm real-time PCR detection of Mycobacterium avium subsp. paratuberculosis" BMC MICROBIOLOGY, BIOMED CENTRAL, LONDON, GB, Bd. 6, Nr. 1, 4. Oktober 2006 (2006-10-04), Seite 87, XP021022432 ISSN: 1471-2180
- TASARA ET AL: "Development and evaluation of a Mycobacterium avium subspecies paratuberculosis (MAP) specific multiplex PCR assay" INTERNATIONAL JOURNAL OF FOOD MICROBIOLOGY, ELSEVIER SCIENCE PUBLISHERS, AMSTERDAM, NL, Bd. 104, Nr. 3, 25. Oktober 2005 (2005-10-25), Seiten 279-287, XP005112462 ISSN: 0168-1605
- TASARA T ET AL: "Development of an F57 sequence-based real-time PCR assay for detection of Mycobacterium avium subsp. paratuberculosis in milk" APPLIED AND ENVIRONMENTAL MICROBIOLOGY, Bd. 71, Nr. 10, Oktober 2005 (2005-10), Seiten 5957-5968, XP002501236 ISSN: 0099-2240
- SCHOENENBRUCHER H ET AL: "New triplex real-time PCR assay for detection of Mycobactetium avium subsp paratuberculosis in bovine feces" APPLIED AND ENVIRONMENTAL MICROBIOLOGY, Bd. 74, Nr. 9, Mai 2008 (2008-05), Seiten 2751-2758, XP002501237 ISSN: 0099-2240

## Beschreibung

Die vorliegende Erfindung betrifft ein Verfahren zum spezifischen und sensitiven Nachweis von Mycobacterium avium ssp. paratuberculosis (MAP) in Organ-; Gewebe- und Kotproben mit einem Real Time-PCR-Verfahren unter Verwendung genomischer Desoxyribonukleinsäure (DNA) eines geeigneten Markers.

### Stand der Technik

*Mycobacterium avium* ssp. *paratuberculosis* (MAP) wurde erstmalig 1895 als ätiologisches Agens bei einer chronisch-entzündlichen Darmerkrankung einer Kuh in Deutschland beschrieben. Der Dedikationsname Johnsche Krankheit/ Johne's Disease wurde für diese bei Wiederkäuern, aber auch weiteren Säugetieren auftretende Paratuberkulose (ParaTB) eingeführt. Es handelt sich um eine Erkrankung, die nahezu weltweit verbreitet ist und zu erheblichen wirtschaftlichen Schäden führt.

Aufgrund der vergleichbaren pathomorphologischen Veränderungen der beim Menschen als Morbus Crohn (MC) bezeichneten chronisch-entzündlichen Darmerkrankung wurde bereits zu Beginn des vorigen Jahrhunderts der Verdacht geäußert, dass solche Mykobakterien ätiologisch eine Rolle im Infektionsgeschehen spielen könnten. Namensgebend war die Beschreibung von acht Fällen einer Ileitis durch Crohn, Ginzburg und Oppenheimer (1932) am Mount Sinai Hospital in New York. Die erste exakte Charakterisierung des klinischen Verlaufs erfolgte bereits 1913 durch Dalziel in Glasgow. Die Entzündung verläuft in Schüben und bedeutet für die Betroffenen eine erhebliche Einschränkung der Lebensqualität. Die Letalität liegt bei ca. 6 %. Eine Multi-Center-Studie für Europa besagt, daß die Inzidenz, d.h. die Neuerkrankungen für MC 5,6 Fälle pro 100 000 Einwohner pro Jahr beträgt; damit sind ca. 200 000 Menschen betroffen.

Die Prävalenz des MC für Deutschland wurde mit 1/500 bis 1/800 angegeben, was in etwa 200.000 Erkrankten entspricht. Die Inzidenz liegt bei 5,2 pro 100.000 Einwohner. Der Gesamtkostenaufwand wird dabei mit 20.000 €/Jahr und Patient angegeben. Dieses summiert sich auf einen Betrag von ca. 2 Mrd. pro Jahr in Deutschland. Die Ähnlichkeiten zwischen klinischen und histologischen Erscheinungen bei der bovinen ParaTB und der humanen intestinalen TB und MC sind nicht zu leugnen, zumal MAP aus Gewebeproben Erkrankter zu isolieren war. Mit der Verbesserung der Nachweismethoden und der Einführung der PCR-Methodik gelang der Nachweis von MAP bei erkrankten Tieren und Menschen immer häufiger.

In der molekularbiologischen Diagnostik diente das Insertionselement "IS*900*" des MAP-Genoms lange als anerkannter Standard. Dabei gehen wissenschaftliche Arbeiten auf die ausschließliche Verwendung von 5 verschiedenen Primerpaaren zurück. Es handelt sich im Einzelnen um IS900/150C//IS900/921, p36//p1, MK5/MK6, P90//P91 und P21/P8. Allerdings werden auch Kreuzreaktionen mit sogenannten "IS*900*like"-Elementen anderer Organismen beschrieben, die diesen Marker für einen spezifischen MAP-Nachweis ungeeignet machen. Mehrere Arbeiten aus dem Stand der Technik beschreiben dennoch die Entwicklung von IS900 gestützten Real Time-PCR Verfahren. Diese Methoden kombinieren die in der konventionellen PCR verwendeten Oligonukleotide mit den aus ELISA-Verfahren bekannten Markerstoffen, z.B. Fluoreszenzfarbstoffen oder radioaktiven Markern, wie z.B. in DE 102 24 338 A1 zum Nachweis von MAP offenbart ist. Allerdings verfügen diese Verfahren nach wie vor nicht über die erforderliche Spezifität zum sicheren Nachweis von MAP und sind anfällig für Kontaminationen oder falsch positive Messergebnisse.

Aus wissenschaftlichen Arbeiten ist ein weiterer Marker für MAP, die Insertionssequenz ISMav2 des MAP-Genoms bekannt. ISMav2 weist zwar keinerlei Ähnlichkeit zu anderen mykobakteriellen Insertionssequenzen auf, jedoch eine über 50%ige Identität zu einem potentiellen Transposon von *Streptomyces coelicolor,* so dass auch hier mögliche Kreuzreaktionen bei der Analyse biologischer Proben nicht ausgeschlossen sind.

Aus DE 10 2005 001 788 A1 ist weiterhin bekannt, dass ein sehr spezifischer Marker für MAP die F57-Sequenz des MAP-Genoms ist, die damit eine geeignete Zielstruktur für selektive PCR-Nachweisverfahren ist.

DE 696 27 625 T2 offenbart, das dnaJ-Gen, ein Gen des Hitzeschockproteins zur Detektion und Bestimmung von Mycobacterien.

Allerdings eignen sich auch F57- und dnaJ-Markersequenz alleine aufgrund von Kreuzreaktionen nicht für einen Einsatz im Real-time PCR-Verfahren zum spezifischen und sicheren Nachweis von MAP in biologischen Proben.

Ein weiterer Nachteil im Stand der Technik ist, dass der Nachweis auf MAP vor allem aus Milch erfolgt, wie z.B. in DE 102 24 338 A1 offenbart. Milch ist eine sehr einfach zu analysierende biologische Probe, mit sehr wenig kontaminierenden Mikroorganismen, die keine PCR-Reaktion hemmenden Inhaltsstoffen aufweist, so dass an die Probenaufbereitung und Durchführung der PCR-Reaktion keine besonderen Anforderungen gestellt werden.

Erforderlich ist jedoch die Analyse weiterer biologischer Proben, wie Kot, Organ- und Gewebeproben, da nicht alle Tierbestände (auch Menschen), die als potentielle Seuchenherde für Paratuberkulose routinemäßig überprüft werden müssen, Milch geben.

Dem Fachmann ist bekannt, dass die Analyse komplexer biologischer Proben, wie Kot, Organ- und Gewebeproben besondere Anforderungen stellt und ein aus der Milchanalytik bekanntes Verfahren nicht unmittelbar übertragen werden kann.

Das Problem der Kreuzreaktion, Neutralisierung und der allgemeinen Kontamination mit anderen Mikroorganismen ist in Kotproben die eine komplexe Zusammensetzung aufweisen, besonders groß. Eine erneute Anreicherung des Erregers in einem Nährmedium ist allgemein unerwünscht, da eine schnelle Diagnose gewährleistet werden muss und die Anzucht von MAP sehr lange dauert.

Es ist derzeit kein Verfahren im Stand der Technik bekannt, dass zufriedenstellend einen automatisierbaren, spezifischen und sicheren Nachweis von MAP in biologischen Proben vor allem Kot-, Organ- und Gewebeproben unter Verwendung von Real-time-PCR offenbart.

### Aufgabe

Aufgabe der vorliegenden Erfindung ist daher es, die beschriebenen Nachteile im Stand der Technik zu beheben und ein verbessertes Real-time-PCR Verfahren zum spezifischen und sicheren Nachweis von MAP in Kot, Organ- und Gewebeproben, sowie einen Testkit, der zur automatisierten Durchführung des Verfahrens geeignet ist, bereitzustellen.

Die Aufgabe wird erfindungsgemäß durch ein Verfahren gemäß der Ansprüche 2-10 und einen Testkit gemäß der Ansprüche 11-16, unter Verwendung spezieller Oligonukleotide gemäß Anspruch 1 gelöst.

Das erfindungsgemäße Verfahren weist mit der Anwendung der Real-time-PCR zahlreiche Vorteile auf, z.B. die Anwendung eines speziellen und sicheren DNA-Extraktionsverfahrens für Kot-, Gewebe- und Organproben, das automatisiert mit einem hohen Probendurchsatz durchgeführt wird. Falsch-negative Reaktionen oder Kreuzreaktionen werden nahezu ausgeschlossen, durch die optimale Auswahl an Oligonukleotiden, als Primer und durch die Amplifizierung von gleichzeitig zwei Markerstrukturen im MAP-Gen. Zusätzlich erfolgt in der Real-time-PCR durch den Vergleich mit einer "Internen Amplifikationskontrolle (IAK)" eine exakte Quantifizierung der amplifizierten spezifischen MAP-DNA. Somit steht ein verbessertes spezifisches und gleichzeitig sensitives Real-time-PCR Verfahren zum Nachweis von *Mycobacterium avium ssp.* paratuberculosis (MAP) zur Verfügung.

Erfindungsgemäß werden optimale Primer für die Amplifizierung der Markersequenzen "ISMav2" und "F57" des MAP-Genoms in einem Real-time-PCR Verfahren zum spezifischen Nachweis von genomischer MAP-DNA aus Kot-, Organ- und Gewebeproben bereitgestellt und kombiniert eingesetzt.

Dabei kommt der vorherigen Aufbereitung des biologischen Materials eine große Bedeutung zu.

Es wird weiterhin ein entsprechender Testkit zur routinemäßigen und automatisierten Durchführung des Verfahrens im Hochdurchsatz bereitgestellt, der die optimalen Primer und alternativ auch die Interne Amplifikationskontrolle (IAK) bzw. Materialien zur Probenaufbereitung und Durchführung des PCR-Verfahrens beinhaltet.

Das erfindungsgemäße Real-time-PCR Verfahren weist selektiv die Existenz von MAP-DNA in Kot-, Organ- und Gewebeproben nach, unabhängig von der untersuchten Tierart. Das Verfahren ermöglicht zusätzlich, im gleichen Verfahrensgang auch die quantitative Bestimmung an nachgewiesener MAP-DNA aus der biologischen Probe. Der spezifische Nachweis erfolgt dabei unabhängig von der vorhandenen Begleitflora der biologischen Proben sowie des Gehaltes an bekannten PCR-Inhibitoren in der Probe. Die MAP-Detektion ist tierartenunabhängig, d. h., dass sowohl MAP-Stämme bovinen, humanen und ovinen Ursprungs zuverlässig nachgewiesen werden. Aufgrund der hohen Sensitivität dieses Verfahrens ist auch bei sehr geringen MAP-Konzentrationen in der biologischen Probe die frühzeitige Erkennung garantiert und es können zu einem sehr frühen Zeitpunkt Maßnahmen ergriffen werden, um einen wesentlichen Beitrag zur Sicherung der Gesundheit von Menschen, aber auch der Tiergesundheit in den Beständen und der Qualität der Lebensmittel zu leisten.

Im Vergleich zu konventionellen PCR-Verfahren, ist das erfindungsgemäße Real-time-PCR-Verfahren wesentlich sensitiver, es werden beispielsweise in Rinderkotproben bis zu 10² Kolonie bildende Einheiten MAP nachgewiesen. Die Nachweisgrenze des Real-time-PCR-Systems, die nach einer dem Fachmann geläufigen Methoden über die Analyse dekadischer Verdünnungsreihen ermittelt wird, liegt bei circa 1,0 x 10⁻¹² g/DNA pro PCR.

Dabei wurde insbesondere der mögliche inhibitorische Einfluß der Hintergrund-DNA aus Kot-, Organ- und Gewebeproben überprüft und keine negativen Auswirkungen auf die Nachweisgrenze festgestellt.

Der wesentliche Vorteil des erfindungsgemäßen Real-time-PCR Verfahrens liegt damit in der um den Faktor 30 höheren Sensitivität im Vergleich zur konventionellen PCR und der direkten Quantifizierung, die eine qualifizierte Diagnose ermöglicht. Dem Fachmann ist zudem ersichtlich, daß durch die zeitauflösenden und parallelen Messungen die arbeits- und zeitintensive gelelektrophoretische Auswertung nicht mehr notwendig ist.

Konventionelle PCR-Verfahren erreichen eine vergleichbare Sensitivität nur durch Verwendung der "Nested-PCR". Diese ist, wie der Fachmann weiß, aufgrund der hohen Kontaminationsgefahr und der damit verbundenen Rate falsch-positiver Ergebnisse jedoch ungeeignet.

Unabhängig von der Probenmatrix und dem vorliegenden Prozessierungs- bzw. Erhitzungsgrad wird die zuverlässige Nachweisführung in Verbindung mit der Probenaufbereitung in weniger als 8 Stunden ("one day result") erreicht.

Zusammen mit den optimierten und für diese Anwendung in der beschriebenen Kombination weiterentwickelten Probenaufbereitungsverfahren wird ein zuverlässiges und sicher arbeitendes Verfahren zur Verfügung gestellt, dessen Durchführung und einfache Auswertung auch von weniger gut ausgebildetem Laborpersonal möglich ist. Die Verfahrensschritte werden auch automatisiert durchgeführt, so dass ein hoher Probendurchsatz erreicht wird.

Das erfindungsgemäße Verfahren zum spezifischen Nachweis von Mycobacterium avium ssp. paratuberculosis (MAP) in Kot-, Gewebe- und Organproben mittels Real-time-PCR, ist charakterisiert durch die Aufbereitung des Proben materials, die DNA-Extraktion und die Analyse der isolierten DNA in der Real-time-PCR, wobei die beiden Markersequenzen ISMav2 und F57des MAP-Genoms mit geeigneten Oligonukleotiden als Primer amplifiziert und mit geeigneten Sonden nachgewiesen werden.

Das Probenaufbereitungsverfahren ist so ausgewählt, dass auch aus komplexen biologischen Proben, wie Kot-, Organ- und Gewebeproben nach Reduzierung des Gehaltes an Hemmstoffen der PCR, mechanischer Homogenisation und einer zuverlässigen Proteinase K-Behandlung, die Gewinnung der gesamten genomischen DNA erfolgt.

Als MAP-spezifische Marker werden erfindungsgemäß die Markersequenzen "ISMav2" und "F57" des MAP-Genoms verwendet, geeignete Oligonukleotide als Vorwärts- und Rückwärtsprimer zur Hybridisierung an diese Gensequenzen hergestellt und zu diesen Primern passende Fluoreszenzsonden ausgewählt.

Die Oligonukleotide als Primer sind mit bekannten Sonden unterschiedlicher Hersteller (z.B. TaqMan, LNA) kompatibel, die an die amplifizierte DNA binden bzw. mit ihr hybridisieren so dass das erfindungsgemäße Real-time-PCR Verfahren geräteunabhängig durchgeführt wird ist und eine breite Anwendbarkeit ermöglicht.

Der mit den erfindungsgemäßen Primern vorzugsweise ausgewählte Genabschnitt zum Nachweis von MAP in biologischen Proben mit Real-time-PCR hat nur eine sehr geringe Größe, er bleibt nachweislich über mehrere Jahre in den Kot-, Organ- und Gewebeproben stabil und ist auch nach längerer Zeit sicher detektierbar.

### Ausführungsbeispiele

### a) Aufbereitung des Probenmaterials und DNA-Extraktion

Als Probenmaterialien, d.h. zu untersuchende Probe oder negative bzw. positive Kontrollprobe werden Kot-, Organ- und Gewebeproben von Menschen, Tieren beispielsweise, Rindern, Geflügel, Schafen oder weiteren Nutz- und Haustieren eingesetzt darunter auch Proben von Lymphknoten oder Darmanteilen. Aufgrund der Vielschichtigkeit der zu untersuchenden Proben, der inhomogenen Verteilung von MAP in der Probe (Klumpenbildung) und der hohen Stabilität der MAP-Zellwand ist der Homogenisierungsschritt wichtig, der so optimiert ist, daß Kontaminationen vermieden werden.

Neben zahlreichen, dem Fachmann bekannten Standardverfahren zur Homogenisierung von biologischen Proben, wie der Verwendung der Schwingmühle, des Elvehjem-Potters, des Ultra Turrax T25, wird vorzugsweise die besonders effiziente und zeitsparende Homogenisation mittels Fast-Prep-120 durchgeführt. Hier besteht der Homogenisationsschritt aus einer Kombination vertikaler Rotations- und horizontaler Auf- und Ab-Bewegungen.

Da das Probenmaterial insbesondere Kot einen hohen Gehalt an Inhibitoren der PCR-Reaktion aufweist (z.B. Gallensäuren, Hämoglobin), werden an die DNA-Extraktion besondere Anforderungen gestellt.

Die Gewinnung der gesamten genomischen DNA aus Kot-, Organ- und Gewebeproben erfolgt bevorzugt nach effizienter Reduzierung des PCR-Hemmstoffgehaltes, dem Einsatz der mechanischen Homogenisation und einer zuverlässigen Proteinase K-Behandlung. Zur Analyse von Kotproben wird beispielsweise z.B. DNeasy^{®} Stool mini Kit von Qiagen, zur Analyse von Organ- und Gewebeproben beispielsweise DNeasy Tissue Kit von Qiagen nach Herstellerangaben verwendet. Zur Reduzierung der dabei auftretenden Schaumbildung wird ein Detergens z.B. DX Puffer hinzugefügt. Zellaufschluss und DNA-Gewinnung erfolgen automatisiert.

Beispielsweise erfolgt die Aufbereitung des Probenmaterials und die DNA-Extraktion aus Kotproben unter folgenden Bedingungen:

Gebrauchslösungen aus DX (Qiagen, Hilden) und ASL-Puffer werden nach Herstellerangaben (Stool Kit) angesetzt: Dazu werden 140 ml Qiagen Puffer ASL auf 70°C erwärmt und 350 µl DX-Puffer zugegeben und gemischt. Der DX-Puffer reduziert die Schaumbildung bei der mechanischen Homogenisation. Danach wird je 1g Kot zu 5 ml DX/ASL-Puffergemisch zugegeben und gevortext. Die Suspension wird für 10 min bei 95°C im Wasserbad inkubiert. Jeweils 1,3 ml des Überstandes werden in je ein 2 ml Plastikgefäß mit Schraubdeckel in das zuvor 4 Glasperlen gegeben wurden, überführt. Der mechanische Aufschluss erfolgt z.B. im Ribolyzer nach folgendem Schema: 4 x 20 sec bei Stufe 6. Anschließend wird für 15 s gevortext. Der gesamte Überstand wird abgenommen und in ein neues 2 ml Gefäß überführt. Um eine deutliche Trennung aus Festphase und Überstand zu erzielen, erfolgt eine Zentrifugation bei 5000xg/5 min. Nach Zugabe einer Inhibtex-Tablette pro Probe zur Bindung der PCR-Hemmstoffe wird für 1 min gevortext. Danach wird erneut für 6 min bei maximaler Geschwindigkeit zentrifugiert. Anschließend wird der gesamte Überstand in ein 1,5 ml Gefäß überführt. Pro Gefäß werden jeweils 20 µl Proteinase K (20 mg/ml) vorgelegt und jeweils 300 µl des Überstandes dazugegeben und gemischt. Anschließend werden jeweils 300 µl AL-Puffer zugefügt und durch 15s Vortexen gemischt. Nach einer 5 minütigen Vorinkubation bei 70°C wird das Gemisch für 10 min bei 95°C inkubiert. Anschlie-βend wird 300 µl Ethanol absolut zugegeben und gut vermischt. Das komplette Lysat wird in zwei Schritten auf die in 2ml-Gefäßen platzierten Säulen überführt. Anschließend wird für 1 min bei 20000*g zentrifugiert. Der Durchfluss wird verworfen und die Säule in ein neues Gefäß gestellt. Zum Waschen werden 500 µl AW1 auf die Säule gegeben, danach wird für 1 min bei 20000xg zentrifugiert. Der Durchfluss wird verworfen und die Säule in ein neues Gefäß gestellt. Zum Waschen werden 500 µl AW2 auf die Säule gegeben, danach wird für 3 min bei 20000g zentrifugiert. Der Durchfluss wird verworfen, die Säule in ein neues Gefäß gestellt und erneut für 1 min bei 20000xg zentrifugiert. Der Durchfluss wird verworfen und die Säule in ein neues 1,5 ml Gefäß gestellt. Anschließend erfolgt die Elution der DNA durch Zugabe von 100 µl AE-Puffer direkt auf die Säule und Inkubation für 1 min bei Raumtemperatur. Nach erneuter Zentrifugation für 1 min bei 20000xg liegt die DNA in der aufgefangenen Flüssigkeit vor und kann direkt für die Real-time-PCR-Analyse verwendet oder bei -20°C gelagert werden.

Alternativ wird statt des DNeasy^{®} Stool mini Kit von Qiagen auch ein anderer Kit verwendet z.B. der High Pure PCR Template Preparation Kit^{®} der Firma Roche Applied Science, der ebenfalls nach Herstellerangaben durchgeführt wird. Alternativ wird als Probenmaterial auch Milch verwendet, die Isolierung der DNA erfolgt dann nach einem Verfahren aus dem Stand der Technik.

### b) Analyse der isolierten DNA in der Real-time-PCR, wobei die beiden Mankersequenzen ISMav2 und F57 amplifiziert und quantifiziert werden

Für die Auswahl der Oligonukleotide als Primer werden die in der NCBI-Gendatenbank hinterlegten Referenzsequenzen für IS*Mav2* (Genbankzugriffs-Nr. AF286339) SEQ ID NO:9 und F57 (Genbankzugriffs-Nr. X70277) SEQ ID NO:10 sowie die Sequenz des MAP-Genoms des Referenzstammes K10 (Genbankzugriffs-Nr. AE016958) verwendet. Die erfindungsgemäß ausgewählten Oligonukleotide binden in einem besonders günstigen Sequenzabschnitt und sind optimal positioniert, um eine selektive Erfassung der beiden MAP-Marker ISMav2 und F57 zu gewährleisten.

Das Amplifikatgrößen (für ISMav2 ca. 120 Basenpaare und F57 circa 60 Basenpaare) sind optimal auf die Erfordernisse der Real-time-PCR-Technologie ausgelegt, für die nach wissenschaftlichen und technischen Statuten eine Produktgröße von 50 bis 150 Basenpaaren gefordert wird. Die geringe Produktgröße ist eine wesentliche Voraussetzung für die Gewährleistung der hohe Sensitivität und Spezifität. Die Sonden sind so ausgewählt, dass sie an die amplifizierte DNA binden und abhängig von der Menge gebundener DNA Signale durch erzeugen, die gemessen und quantifiziert werden. Bevorzugt tragen die Sonden einen Fluoreszenzfarbstoff, so dass ihre Fluoreszenz gemessen wird.

**Tabelle 1**

| **Nomenklatur** | **Nukleotidsequenz** | **Binde-Position** | **Basenanzahl** |
|---|---|---|---|
| **SEQ ID NO:1 Vorwärtsprimer IS*Mav2*** | 5'-CGG CAA AAT CGA GCA GTT TC-3' | 1645-1665 *ISMav2* | 20 |
| **SEQ ID NO:2 Rückwärtsprimer IS*Mav2*** | 5'-TGA GCC GGT GTG ATC ATC TTT-3' | 1786-1807 *ISMav2* | 21 |
| **SEQ ID NO:3 Ta-qMan_{mgb}-Sonde IS*Mav2*** | FAM-CGC TGA GTT CCT TAG-MGB | 1678-1693 *ISMav2* | 15 |
| **SEQ ID NO:4 LNA-Sonde IS*Mav2*** | FAM-cGc tGa GtT cCt TaG- BHQ1 | 1678-1693 IS*Mav2* | 15 |
| | | | |
| **SEQ ID NO:5 Vorwärtsprimer F57** | 5'-TAC GAG CAC GCA GGC ATT C-3' | 244-263 F57 | 19 |
| **SEQ ID NO:6 Rückwärtsprimer F57** | 5'-CGG TCC AGT TCG CTG TCA T-3' | 288-307 F57 | 19 |
| **SEQ ID NO:7 Ta-qMan_{mgb}-Sonde F57** | VIC-CCT GAC CAC CCT TC-MGB | 268-282 F57 | 14 |
| **SEQ ID NO:8 LNA-Sonde F57** | Yakima Yellow-cCtgAcCacCctTc-BHQ1 | 268-282 F57 | 14 |

Tabelle 1 zeigt die Nukleinsäuresequenzen der erfindungsgemäßen Primer zur Analyse der DNA in der Real-time-PCR und die Nukleinsäuresequenzen der Sonden TaqMan_{mgb}-Sonde bzw. LNA-Sonde.

Die erfindungsgemäßen Primer sind Nukleinsäuremoleküle, die an die ISMav2- bzw. F57-Markersequenz binden bzw. hybridisieren, dabei ist dem Fachmann klar, dass auch abweichend Derivate der Primer, die durch Deletion, Substitution und/oder Insertion entstanden sein und vorzugsweise mindestens eine 80 %ige Homologie zu den dargestellten Primern aufweisen davon umfasst sind.

Die Sequenzen der erfindungsgemäßen Primer zur Analyse der DNA in der Real-time-PCR und die der Sonden TaqMan_{mgb}-Sonde bzw. LNA-Sonde setzen sich aus folgenden Nukleinsäuresequenzen zusammen:

Erläuterungen zur Nomenklatur für die Primer- und Sondenkombinationen:
- TaqMan^{®} ist ein eingetragenes Warenzeichen der Fa. Hoffman-La Roche.
- Die Bezeichnung mgb/MGB steht für minor groove binder und bezeichnet eine dem Fachmann geläufige Methode des Sondendesigns mit der eine höhere Bindungsaffinität zur Zielregion und die Auswahl von Sonden mit einer kürzeren Basensequenz ermöglicht wird.
- LNA-Sonde steht für Locked Nucleic Acids (LNAs), sie werden in doppelt farbstoffmarkierte Sonden integriert. Da LNA-Basen, die Schmelztemperatur merklich erhöhen, sind zweifach farbstoffmarkierte LNA-Sonden kürzer als herkömmliche DNA-Sonden (< 50 Basenpaare). Kürzere Sonden weisen einen besseren ,Quenching'-Effekt auf, haben ein geringeres Hintergrundrauschen und sind daher sensitiver.
- FAM bezeichnet eine dem Fachmann geläufige, für die Fluoreszenzfarbstoffmarkierung von Sonden gewählte Substanz, die alternativ auch als Fluörescein bezeichnet wird.
- VIC bezeichnet ebenfalls eine dem Fachmann geläufige, für die Fluoreszenzfarbstoffmarkierung von Sonden gewählte Substanz. Der Einsatz weiterer Flurophore entspricht dem technischen Standard und ist dem Fachmann geläufig.

Die erfindungsgemäßen TaqMan_{mgb}-Sonden bzw. LNA-Sonden werden jeweils in Kombination mit dem jeweiligen Primerpaar als Duplex-Real-time-PCR eingesetzt. In Verbindung mit einer Internen Amplifikationskontrolle (IAK) erfolgt die Anwendung als sog. Triplex-Real-time-PCR. Als Analysegerät wird beispielsweise das ABI 7000 Sequence Detection System (Fa. Applied Biosystems, Darmstadt) genutzt, alternativ auch andere Geräte der Fa. Applied Biosystems, sowie alle weiteren bekannten Geräte, die das TaqMan-Prinzip verwenden (BioRad Laboratories, Stratagene), sowie der Light Cycler V.2.0 der Fa. Roche.

Kombiniert werden zum Nachweis der ISMav2-Markersequenz der Vorwärtsprimer ISMav2 SEQ ID NO:1 und der Rückwärtsprimer ISMav2 SEQ ID NO:2 mit TaqMan_{mgb}-Sonde ISMav2 SEQ ID NO:3 oder alternativ Vorwärtsprimer ISMav2 SEQ ID NO:1 und Rückwärtsprimer ISMav2 SEQ ID NO:2 mit LNA-Sonde ISMav2. SEQ ID NO:4.

Kombiniert werden zum Nachweis der F57- Markersequenz der Vorwärtsprimer F57 SEQ ID NO:5 und Rückwärtsprimer F57 SEQ ID NO:6 mit TaqMan_{mgb}-Sonde F57 SEQ ID NO:7 oder alternativ Vorwärtsprimer F57 SEQ ID NO:5 und Rückwärtsprimer F57 SEQ ID NO:6 mit LNA-Sonde F57 SEQ ID NO:8.

Zur Quantifizierung wird DNA eines MAP-Stammes (z. B. des bovinen Stammes "423") gewonnen und die DNA-Konzentration bestimmt. Aus diesem wird eine dekadische Verdünnungsreihe in der jeweiligen Real Time-PCR eingesetzt. Dabei wird die Konzentration der gewonnenen DNA via UV-Spektroskopie bestimmt. Anschließend wird im Rahmen der Konfiguration des verwendeten Real Time-Cyclers die dekadische Verdünnungsreihe als Standard definiert und die spektroskopisch ermittelten Werte manuell als Berechnungsgrundlage in die Bedienungssoftware des Thermocyclers eingegeben. Der DNA-Gehalt der zu untersuchenden Proben wird als Relation zu den im gleichen PCR-Lauf mitgeführten Standards ermittelt.

Folgende Mastermixkonditionen (50 µl-Ansatz) werden bevorzugt eingesetzt:

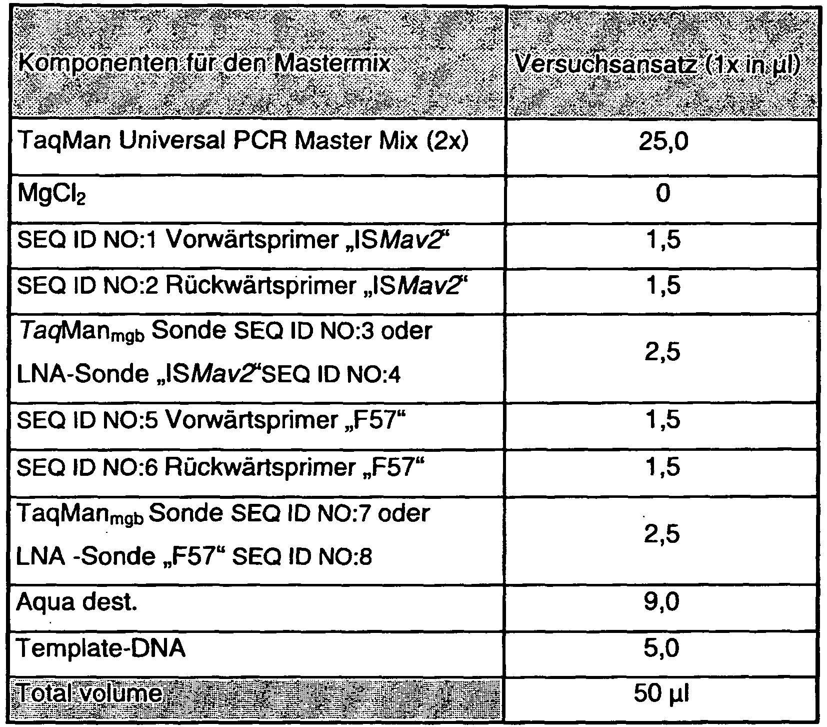

Template-DNA ist z. B. die aus der biologischen Probe nach Schritt a) gewonnene DNA oder eine MAP-positive bzw. MAP-negative Kontrollprobe.

Die verwendeten Reagenzien sind als Komplettformulierung im TaqMan-Universal Mastermix (Fa. Applied Biosystems, Darmstadt) enthalten. Alternativ werden andere Reagenzien, z. B. der Fa. Eurogentec oder Qiagen eingesetzt.

Die Thermocylclerkonditionen werden wie folgt gewählt:

Initale UNG-Inkubation bei 50°C für 2 min, Aktivierung der Polymerase z.B. AmpliTaq Gold Polymerase (Fa. Applied Biosystems, Darmstadt) bei 95°C. Der Analysezeitraum beträgt 50 Zyklen, es folgt die Denaturierung bei 95°C für 15 s und die Annealingphase bei 60°C für 1 min. Jede Messung erfolgt alternativ im Duplikatansatz. Die Datenauswertung, besteht aus Festlegung des Schwellenwertzyklusses (Cₜ-Wert) und Bestimmung des quantitativen MAP-DNA-Gehaltes [Einheit: ng/µl] in der untersuchten Probe. Diese wird nach den dem Fachmann geläufigen Methoden vorgenommen.

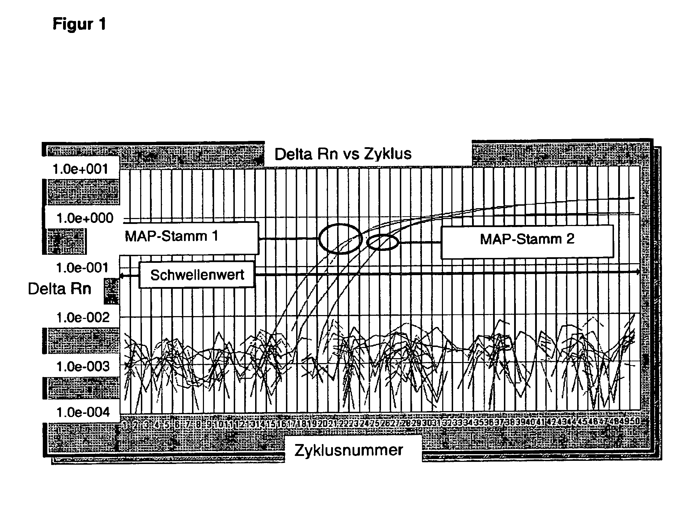

Die abschließende Auswertung der Ergebnisse erfolgt nach der dem Fachmann geläufigen Vorgehensweise, die exemplarisch in Figur 1 für die Darstellung der spezifischen Amplifikation von MAP gezeigt ist.

Die elektronische Datenerfassung und Dokumentation erfolgt nach den Vorgaben im Rahmen der diagnostischen Qualitätssicherung für die moderne Laborpraxis.

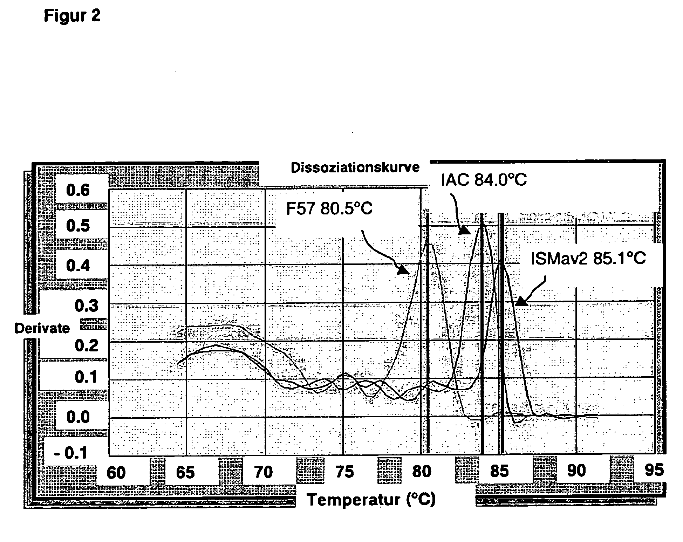

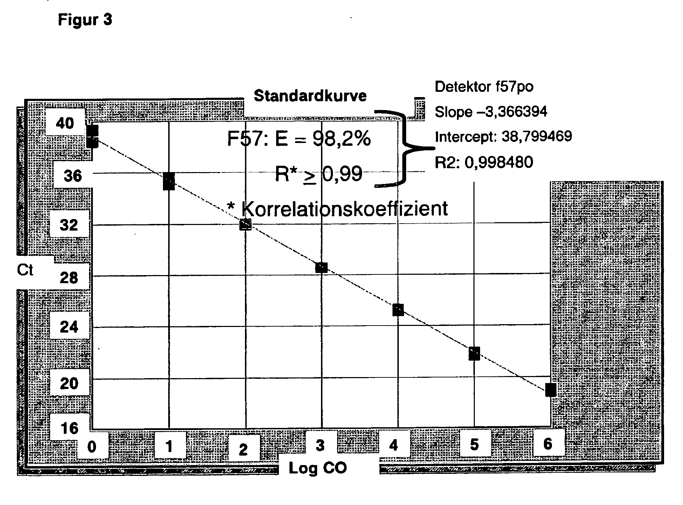

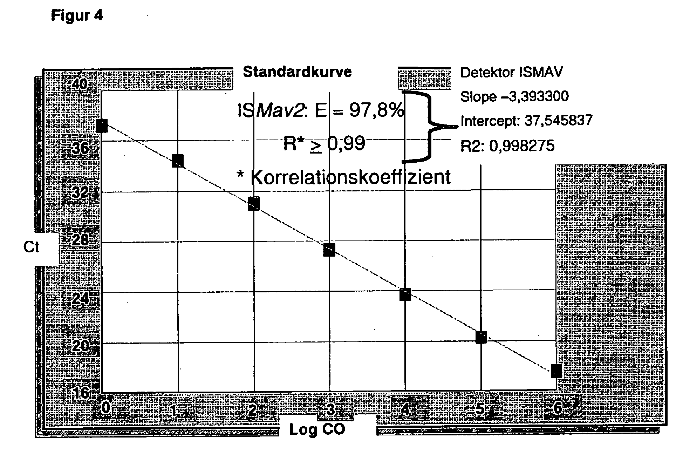

Figur 2 zeigt die Schmelzkurvenanalyse. Figur 3 und 4 zeigen die Amplifikationseffizienz.

Zur Validierung des Verfahrens führten Ergebnisse mit 49 MAP-, 34 Nicht-MAP-, 65 Nicht-Mycobacterium-Referenz- und Feldstämmen.

Eine Ausführungsform der Erfindung betrifft einen einfach zu handhabenden Testkit zum spezifischen und sensitiven Nachweis von MAP in Gewebe- und Kotproben, mindestens enthaltend:

| |
|---|
| **SEQ ID NO:1** Vorwärtsprimer "IS*Mav2*" |
| **SEQ ID NO:2** Rückwärtsprimer "IS*Mav2*" |
| **SEQ ID NO:3** TaqMan_{mgb} oder |
| **SEQ ID NO:4** LNA-Sonde "IS*Mav2*" |
| **SEQ ID NO:5** Vorwärtsprimer "F57" |
| **SEQ ID NO:6** Rückwärtsprimer "F57" |
| **SEQ ID NO:7** TaqMan_{mgb} oder |
| **SEQ ID NO:8** LNA -Sonde "F57" |

Der Testkit zum spezifischen Nachweis von Mycobacterium avium ssp. paratuberculosis (MAP) in Kot-, Gewebe - und Organproben mittels Real-Time PCR umfasst Oligonukleotide als Primer zur Amplifizierung von MAP-DNA gemäß SEQ ID NO:1, SEQ ID NO:2 und SEQ ID NO:5, SEQ ID NO:6, die mit Gensequenzen für ISMav2 und F57 des MAP-Genoms hybridisieren. Alternativ umfasst der Testkit Sonden, die an die amplifizierte DNA binden und abhängig von der Menge gebundener DNA Signale erzeugen, die gemessen und quantifiziert werden. Vorzugsweise sind diese Sonden mit einem Fluoreszenzfarbstoff gekoppelt und weisen SEQ ID NO:3 und SEQ ID NO:7 auf oder alternativ SEQ ID NO:4 und SEQ ID NO:8.

In einer bevorzugten Ausführungsform umfasst der Testkit weiterhin Reagenzien zur Gewinnung der gesamten genomischen DNA aus Kot-, Organ- und Gewebeproben, wobei die Reagenzien den Gehalt an Hemmstoffen der PCR reduzieren und Proteinase K enthalten.

Zusätzlich enthält der Test alternativ eine Interne Amplifikationskontrolle, die eine definierte Menge an MAP-DNA enthält.

Der Testkit schließt aber auch die Formulierung als Einzeldetektionssystem unter Verwendung der Oligonukleotide zur Detektion von "ISMav2" oder "F57" ebenso ein, wie die Auslegung als Multiplex-PCR.

Auf Grund der Lehre der vorliegenden Erfindung sowie auf Grund des allgemeinen Fachwissens in diesem technischen Gebiet ist dem Hersteller des erfindungsgemäßen Kits bekannt, wie er die einzelnen Komponenten des Kits, z.B. Puffer herstellt, formuliert und lagert.

### Abbildungen

Fig. 1
   Spezifische Amplifikation zweier boviner MAP-Stämme MAP-Stamm 1 und MAP-Stamm 2 mit den Markern "ISMav2" und "F57"
Fig. 2
   Dissoziationskurve und Angabe des Schmelzpunktes für Markersequenz "ISMav2" bei 84,0 °C, für Markersequenz "F57" bei 80,5 °C und für die internen Amplifikationskontrolle (IAK) bei 85,1 °C
Fig. 3
   Amplifikationseffizienz von "F57" in der Triplex-Real-Time-PCR mit einem Wert: E=98,2 %
Fig. 4
   Amplifikationseffizienz von "IS*Mav2*" in der Triplex-Real-Time-PCR mit einem Wert: E = 97,8°C

## Patentansprüche

1. Satz aus Oligonukleotiden zur Verwendung bei einem spezifischen Nachweis von *Mycobacterium avium ssp. paratuberculosis* (MAP) in Kot-, Gewebe- und Organproben mittels Real-time-PCR, wobei die Oligonukleotide folgende Nukleotidsequenzen aufweisen:
SEQ ID NO:1 Vorwärtsprimer ISMav2 5'-CGG CAA AAT CGA GCA GTT TC-3'
SEQ ID NO:2 Rückwärtsprimer ISMav2 5'-TGA GCC GGT GTG ATC ATC TTT-3'
SEQ ID NO:5 Vorwärtsprimer F57 5'-TAC GAG CAC GCA GGC ATT C-3'
SEQ ID NO:6 Rückwärtsprimer F57 5'-CGG TCC AGT TCG CTG TCA T-3'

2. Verfahren zum spezifischen Nachweis von *Mycobacterium avium ssp. paratuberculosis* (MAP) in Kot-, Gewebe - und Organproben mittels Real-time-PCR, **dadurch gekennzeichnet, dass** die Markersequenzen ISMAV2 SEQ ID NO:9 und F57 SEQ ID NO:10 des MAP-Genoms nachgewiesen werden und die Oligonukleotide SEQ ID NO:1, SEQ ID NO:2, SEQ ID NO:5 und SEQ ID NO:6 gemäß Anspruch 1 eingesetzt werden.

3. Verfahren gemäß Anspruch 2, **dadurch gekennzeichnet, dass** die Kot-, Gewebe- und Organproben von Rindern, Schweinen, Geflügel oder Menschen stammen.

4. Verfahren gemäß Anspruch 2 und 3, **dadurch gekennzeichnet, dass** zur Aufbereitung der Kot-, Gewebe- und Organproben Reagenzien verwendet werden, die den Gehalt an Hemmstoffen der PCR reduzieren und Proteinase K enthalten.

5. Verfahren gemäß Anspruch 2 bis 4, **dadurch gekennzeichnet, dass** aus den Kot-, Gewebe- und Organproben DNA isoliert wird.

6. Verfahren gemäß Anspruch 2 bis 5, **dadurch gekennzeichnet, dass** die Real-time-PCR in Gegenwart einer Internen Amplifikationskontrolle durchgeführt wird.

7. Verfahren gemäß Anspruch 2 bis 6, **dadurch gekennzeichnet, dass** die Interne Amplifikationskontrolle eine definierte Menge an MAP-DNA enthält.

8. Verfahren gemäß Anspruch 2 bis 7, **dadurch gekennzeichnet, dass** die Real-time-PCR in Gegenwart von Sonden durchgeführt wird, die an die amplifizierte DNA binden und abhängig von der Menge gebundener DNA Signale erzeugen, die gemessen und quantifiziert werden.

9. Verfahren gemäß Anspruch 8, **dadurch gekennzeichnet, dass** als Sonde TaqMan_{mgb} gemäß SEQ ID NO:3 und SEQ ID NO:7 oder LNA-Sonde gemäß SEQ ID NO:4 und SEQ ID NO:8 verwendet wird.

10. Verfahren gemäß Anspruch 9, **dadurch gekennzeichnet, dass** die Sonden mit Fluoreszenzfarbstoff gekoppelt sind.

11. Testkit zum spezifischen Nachweis von *Mycobacterium avium ssp. paratuberculosis* (MAP) in Kot-, Gewebe- und Organproben mittels Real-time-PCR, **dadurch gekennzeichnet, dass** der Testkit Oligonukleotide zur Amplifizierung von MAP-DNA gemäß Anspruch 1 umfasst, die mit Gensequenzen für ISMav2 und F57 des MAP-Genoms hybridisieren.

12. Testkit gemäß Anspruch 11, **dadurch gekennzeichnet, dass** er Sonden umfasst, die an die amplifizierte DNA binden und abhängig von der Menge gebundener DNA Signale erzeugen, die gemessen und quantifiziert werden.

13. Testkit gemäß Anspruch 11 und 12, **dadurch gekennzeichnet, dass** er die Sonde TaqMan_{mgb} gemäß SEQ ID NO:3 und SEQ ID NO:7 oder LNA-Sonde gemäß SEQ ID NO:4 und SEQ ID NO:8 umfasst.

14. Testkit gemäß Anspruch 11 bis 13, **dadurch gekennzeichnet, dass** die Sonden mit Fluoreszenzfarbstoff gekoppelt sind.

15. Testkit gemäß Anspruch 11 bis 14, **dadurch gekennzeichnet, dass** er Reagenzien zur Gewinnung der gesamten genomischen DNA aus Kot-, Organ- und Gewebeproben umfasst, wobei die Reagenzien, den Gehalt an Hemmstoffen der PCR reduzieren und Proteinase K enthalten.

16. Testkit gemäß Anspruch 11 bis 13, **dadurch gekennzeichnet, dass** er eine Interne Amplifikationskontrolle aufweist, die eine definierte Menge an MAP-DNA enthält.

## Claims

1. Set of oligonucleotides for use in a specific identification of *Mycobacterium avium ssp. paratuberculosis* (MAP) in fecal, tissue and organ samples using real-time PCR, wherein the oligonucleotides comprise the following nucleotide sequences:
SEQ ID NO:1 forward primer IS*Mav2* 5'-CGG CAA AAT CGA GCA GTT TC-3'
SEQ ID NO:2 reverse primer IS*Mav2* 5'-TGA GCC GGT GTG ATC ATC TTT-3'
SEQ ID NO:5 forward primer F57 5'-TAC GAG CAC GCA GGC ATT C-3'
SEQ ID NO:6 reverse primer F57 5'-CGG TCC AGT TCG CTG TCA T-3'

2. Method for the specific identification of *Mycobacterium avium ssp. paratuberculosis* (MAP) in fecal, tissue and organ samples using real-time PCR, **characterized in that** the marker sequences ISMAV2 SEQ ID NO:9 and F57 SEQ ID NO:10 of the MAP genome are identified and the oligonucleotides SEQ ID NO:1, SEQ ID NO:2, SEQ ID NO:5 and SEQ ID NO:6 according to claim 1 are applied.

3. Method according to claim 2, **characterized in that** the fecal, tissue and organ samples originate from cattle, swine, poultry or humans.

4. Method according to claims 2 and 3, **characterized in that** reagents are used for the preparation of the fecal, tissue and organ samples that reduce the content of PCR inhibitors and contain proteinase K.

5. Method according to claims 2 to 4, **characterized in that** DNA is isolated from the fecal, tissue and organ samples.

6. Method according to claims 2 to 5, **characterized in that** the real-time PCR is carried out in the presence of an internal amplification control.

7. Method according to claims 2 to 6, **characterized in that** the internal amplification control contains a defined quantity of MAP-DNA.

8. Method according to claims 2 to 7, **characterized in that** the real-time PCR is carried out in the presence of probes, which bind to the amplified DNA and, depending on the quantity of bound DNA, generate signals that are measured and quantified.

9. Method according to claim 8, **characterized in that** *Taq*Man_{mgb} according to SEQ ID NO:3 and SEQ ID NO:7 or an LNA probe according to SEQ ID NO:4 and SEQ ID NO:8 is used as the probe.

10. Method according to claim 9, **characterized in that** the probes are coupled to fluorescent dye.

11. Test kit for the specific identification of *Mycobacterium avium ssp. paratuberculosis* (MAP) in fecal, tissue and organ samples using real-time PCR, **characterized in that** the test kit includes oligonucleotides for the amplification of MAP-DNA according to claim 1 that hybridize with gene sequences for ISMav2 and F57 of the MAP genome.

12. Test kit according to claim 11, **characterized in that** it comprises probes that bind to the amplified DNA and, depending on the quantity of bound DNA, generate signals that are measured and quantified.

13. Test kit according to claims 11 and 12, **characterized in that** it includes the probe TaqMan_{mgb} according to SEQ ID NO:3 and SEQ ID NO:7 or an LNA probe according to SEQ ID NO:4 and SEQ ID NO:8.

14. Test kit according to claims 11 to 13, **characterized in that** the probes are coupled to fluorescent dye.

15. Test kit according to claims 11 to 14, **characterized in that** it comprises reagents for the extraction of the entire genomic DNA from fecal, organ and tissue samples, the reagents reducing the content of PCR inhibitors and containing proteinase K.

16. Test kit according to claims 11 to 13, **characterized in that** it comprises an internal amplification control which contains a defined quantity of MAP-DNA.

## Revendications

1. Ensemble d'oligoucléotides pour l'utilisation lors d'une détection spécifique de *Mycobacterium avium ssp. paratuberculosis* (MAP) dans des échantillons de selles, de tissus et d'organes, à l'aide d'une PCR en temps réel, les oligonucléotides présentant les séquences nucléotidiques suivantes :
SEQ ID NO:1 amorce sens IS*Mav*2 5'-CGG CAA AAT CGA GCA GTT TC-3'
SEQ ID NO:2 amorce antisens IS*Mav2* 5'-TGA GCC GGT GTG ATC ATC TTT-3'
SEQ ID NO:5 amorce sens F57 5'-TAC GAG CAC GCA GGC ATT C-3' SEQ ID NO:6 amorce antisens F57 5'-CGG TCC AGT TCG CTG TCA T-3'

2. Procédé d'identification spécifique de *Mycobacterium avium ssp. paratuberculosis* (MAP) dans des échantillons de selles, de tissus et d'organes à l'aide d'une PCR en temps réel, **caractérisé en ce que** l'on détecte les séquences de marqueur ISMAV2 SEQ ID NO:9 et F57 SEQ ID NO:10 du génome de MAP sont détectées et on utilise les oligonucléotides SEQ ID NO:1, SEQ ID NO:2, SEQ ID NO:5 et SEQ ID NO:6 selon la revendication 1.

3. Procédé selon la revendication 2, **caractérisé en ce que** les échantillons de selles, de tissus et d'organes, proviennent de bovins, de porcs, de volaille et d'êtres humains.

4. Procédé selon l'une des revendications 2 et 3, **caractérisé en ce que**, pour la préparation des échantillons de selles, de tissus et d'organes, on utilise des réactifs qui réduisent la teneur en inhibiteurs de PCR et qui contiennent la protéinase K.

5. Procédé selon l'une des revendications 2 à 4, **caractérisé en ce que** l'ADN est isolé à partir des échantillons de selles, de tissus et d'organes.

6. Procédé selon l'une des revendications 2 à 5, **caractérisé en ce que** le PCR en temps réel est mis en oeuvre en présence d'un témoin interne d'amplification.

7. Procédé selon l'une des revendications 2 à 6, **caractérisé en ce que**, le témoin interne d'amplification contient une quantité définie d'ADN de MAP.

8. Procédé selon l'une des revendications 2 à 7, **caractérisé en ce que** la PCR en temps réel est réalisée en présence de sondes qui se lient à l'ADN amplifié et, en fonction de la quantité d'ADN lié, produisent des signaux qui sont mesurés et quantifiés.

9. Procédé selon la revendication 8, **caractérisé en ce qu'**on utilise, en tant que sonde, une sonde TaqMan_{mgb} selon SEQ ID NO:3 et SEQ ID NO:7 ou une sonde LNA selon ID SEQ ID NO:4 et SEQ ID NO:8.

10. Procédé selon la revendication 9, **caractérisé en ce que** les sondes sont couplées à un colorant fluorescent.

11. Kit de test pour l'identification spécifique de *Mycobacterium avium ssp. paratuberculosis* (MAP) dans des échantillons de selles, de tissus et d'organes, à l'aide d'une PCR en temps réel, **caractérisé en ce que** le kit de test comprend des oligonucléotides pour l'amplification d'ADN de MAP selon la revendication 1, qui s'hybrident avec des séquences de gène pour ISMav2 et F57 du génome de MAP.

12. Kit de test selon la revendication 11, **caractérisé en ce qu'** il contient des sondes, qui se lient à l'ADN amplifié et produisent, en fonction de la quantité d'ADN lié, des signaux qui sont mesurés et quantifiés.

13. Kit de test selon l'une des revendications 11 et 12, **caractérisé en ce qu'**il contient la sonde TaqMan_{mgb} selon SEQ ID NO:3 et SEQ ID NO:7 ou la sonde LNA selon SEQ ID NO:4 et SEQ ID NO:8.

14. Kit de test selon l'une des revendications 11 à 13, **caractérisé en ce que** les sondes sont couplées à un colorant fluorescent.

15. Kit de test selon l'une des revendications 11 à 14, **caractérisé en ce qu'** il contient des réactifs pour la préparation d'ADN génomique entier provenant d'échantillons de selles, d'organes et de tissus, les réactifs réduisant la teneur en inhibiteurs de PCR et comprenant de la protéinase K.

16. Kit de test selon l'une des revendications 11 à 13, **caractérisé en ce qu'**il présente un témoin interne d'amplification, qui contient une quantité définie d'ADN de MAP.


### SEQUENCE LISTING

<110> Justus-Liebig-Universität Giessen
<120> verfahren zum Nachweis von Paratuberkulose
<130> TM203
<150> DE 10 2007 015 775.6 <151> 2007-03-30
<160> 10
<170> PatentIn version 3.4
<210> 1
   <211> 20
   <212> DNA
   <213> Artificial sequence
<220>
   <223> Primer for Real-time-PCR
<220>
   <221> SEQ ID No:1 vorwärtsprimer ISMav2
   <222> (1)..(20)
   <223> Determination of MAP ISMav2-sequence
   Bindingposition 1645 to 1665 at the ISMav2-sequence
<400> 1
   cggcaaaatc gagcagtttc 20
<210> 2
   <211> 21
   <212> DNA
   <213> artificial sequence
<220>
   <223> Primer for Real-time-PCR
<220>
   <221> SEQ ID NO:2 Rückwärtsprimer ISMav2
   <222> (1)..(21)
   <223> Identification of MAP IsMav2-sequence
   Bindingposition 1786 to 1807 at the ISMav2-sequence
<400> 2
   tgagccggtg tgatcatctt t 21
<210> 3
   <211> 15
   <212> DNA
   <213> Mycobacterium avium subsp. paratuberculosis
<220>
   <221> SEQ ID NO:3 TaqManmgb-Sonde ISMav2
   <222> (1)..(15)
   <223> Identification of MAP ISMav2-sequence
   Bindingposition 1678 to 1693 at the ISMav2-sequence
<400> 3
   cgctgagttc cttag 15
<210> 4
   <211> 15
   <212> DNA
   <213> Mycobacterium avium subsp. paratuberculosis
<220>
   <221> SEQ ID NO:4 LNA-Sonde ISMav2
   <222> (1)..(15)
   <223> Identification of MAP ISMav2 Sequence
   Bindingposition 1678 to 1693 at the ISMav2-sequence
<400> 4
   cgctgagttc cttag 15
<210> 5
   <211> 19
   <212> DNA
   <213> artificial sequence
<220>
   <223> Primer for Real-time-PCR
<220>
   <221> SEQ ID NO:5 vorwärtsprimer F57
   <222> (1)..(19)
   <223> Identification of MAP F57 sequence
   Bindingposition 244 to 263 at the F57-sequence
<400> 5
   tacgagcacg caggcattc 19
<210> 6
   <211> 19
   <212> DNA
   <213> artificial sequence
<220>
   <223> Primer for Real-time-PCR
<220>
   <221> SEQ ID NO:6 Rückwärtsprimer F57
   <222> (1)..(19)
   <223> Identification of MAP F57 sequence
   Bindingposition 288 to 307 at the F57-sequence
<400> 6
   cggtccagtt cgctgtcat 19
<210> 7
   <211> 14
   <212> DNA
   <213> Mycobacterium avium subsp. paratuberculosis
<220>
   <221> SEQ ID NO:7 TaqManmgb-Sonde F57
   <222> (1)..(14)
   <223> Identification of MAP F57 sequence
   Bindingposition 268 to 282 at the F57-sequence
<400> 7
   cctgaccacc cttc 14
<210> 8
   <211> 14
   <212> DNA
   <213> Mycobacterium avium subsp. paratuberculosis
<220>
   <221> SEQ ID NO:8 LNA-Sonde F57
   <222> (1)..(14)
   <223> Identification of MAP F57 sequence
   Bindingposition 268 to 282 at the F57-sequence
<400> 8
   cctgaccacc cttc 14
<210> 9
   <211> 2263
   <212> DNA
   <213> Mycobacterium avium subsp. paratuberculosis
<220>
   <221> SEQ ID NO:9 ISMav2 putative transposase gene
   <222> (1)..(2263)
   <223> Position 1645 to 1665 for binding of vorwärtsprimer ISMav2
   Position 1786 to 1807 for binding of Rückwärtsprimer ISMav2
   Position 1678 to 1693 for binding of TaqMan-Sonde ISMav2 or LNA-Sonde ISMav2
<300>
   <308> AF286339
   <309> 2005-05-11
   <313> (1)..(2263)
<400> 9
   gagctcggcc agcacttcat ccgggtgaat tccgtgcacc ccaccaacgt gaacacaccg 60
   atgttcatga acgaggggac gatgaggctg ttccggccgg acctgaagaa ccccggcccg 120
   gacgacctga aggtcgccgc gcagttcatg catgtgctgc cggtcggctg ggtggagccg 180
   gtggacatca gcaacgccgt gctgttcctg gcctccgacg aatcgcgtta catcacaggt 240
   cttccggtca ccctcgacgc cggcagcatg ctcaagtagc cgcgccggca tctcgctcac 300
   cggcggtgag gatcatcccg gtggccaggg gcgggatcga accgccgacc ttccgctttt 360
   caggcggacg ctcgtaccga ctgagctacc tggccggaag gcagcgagtg gctcgccgcg 420
   ctggcgaccc tgacgggact cgaacccgcg acctccgccg tgacagggcg gcgcgctaac 480
   caactgcgcc acagggcctt gctgctgctc cgcgtcgccg cgttgcgtac cccctacggg 540
   attcgaaccc gcgctaccgc cttgaaaggg cggcgtccta ggccgctaga cgaagggggc 600
   cagaaccgaa tctctccggg gtactcgcaa cgtggtttcg ttgggagcca cgtcagctta 660
   ggtcaccgtg ggcccaatcc tcaaacgagc ccggttttgg gtccaagtat cctgaaactc 720
   cgcggcccct atagctcagt tggtagagct acggactttt aatccgcagg tcctaggttc 780
   gagtcctagt gggggcacca gatgtatgta tgacgtcggc tgatgtgtga cgttttgtct 840
   tcggttgatg tgtgacggtg tttcggttga tgcgtgacag ttgtttcggc tgatcggtga 900
   cactccctag atgagggagt taagtgtggc tgagcagcgg tatcaggccg tgatggcggt 960
   aatcagtgac gggttgtcgg tgtcgcaggc cgcagagaag ttcggggtgg cgcgtcagac 1020
   gctgcaccga tggctggccc ggtatgaagc cgcgggcctg aaggggctgg tggatcggtc 1080
   gcatcggccg gtgagttgtc cgcatcagat gctggcggta gtggaggcgg cggtgttgga 1140
   gttgcgccgc tcgcggccct attgggggcc gcggcggttg gtgttcgagt tggccaagcg 1200
   aggtgtccat ccggtgccgt cggagtcgcc ggtgtatcga gcgctggtgc gggccggtct 1260
   gatcgacccc gcgatgcgag atcgacggtc gcgcaaatgg aagcgctggg agcgcggggc 1320
   gccgatggag ttgtggcagc tcgacatcgt cggcgggttc ccgctggccg atgggaccag 1380
   cgccaaagcc ctgaccggca tcgatgatca ttcccggatg tgtgtgtgcg ccaagctgat 1440
   ggcccgtgag cgcacccgcg cggtctgcga cggattacgg gcggcgctgg ccgcttacgg 1500
   ggtgcctgag cagatcttga ccgataacgg caaggtgttc accgagcggt tttgtcatcc 1560
   accggtcgag gtgctctttg atgcgatctg ccgcgagcac ggcatcgaac atctgttgac 1620
   ccagccgcgc agcccgacca cgaccggcaa aatcgagcag tttcaccgca gtctgcgcgc 1680
   tgagttcctt agcggccgtg agcctttcac caacctcaag gtcgctcagc aggcgctcga 1740
   tgagtgggtc gaggactaca acaccacccg gccgcaccaa gccctaaaga tgatcacacc 1800
   ggctcaacgg tttcacgccg gtgcgccggc atcaccaccg tcgaactcgt gtgcccgaca 1860
   cgtcgaccgc agtggtgatg actgggtgag tcggcgggtg tgctccaacg gcatcgtgtg 1920
   cgtgtcctgg cagcaggtct gtatcgggcg ccactatgcc ggcgcccgct gcgatgtcca 1980
   cgtcgatggg gacctgctga ggttttgggt cggcgacaat ctggtcaaaa ccgccgcgcg 2040
   caccagccgc ggcgaggtac gaaacaaaca ggccctgcgc accaacgcac cggcctaaaa 2100
   cacaacccag agtgtcaccg atcaaccgac atagaaatgt caccgagcaa ccgaccctga 2160
   acaggggcac cagagagcag ccccgcccgc tcaggtggtc gacgatgtgg ctgcgctcgc 2220
   cgtggtagac caggtcattg aaatcgacct cgaacccgag ctc 2263
<210> 10
   <211> 620
   <212> DNA
   <213> M.paratuberculosis species specific F57 DNA fragment
<220>
   <221> SEQ ID NO:10 M.paratuberculosis species specific F57 DNA fragment
   <222> (1)..(620)
   <223> Position 244 to 263 for binding of vorwärtsprimer F57
   Position 288 to 307 for binding of Rückwärtsprimer F57
   Position 268 to 282 for binding of TaqMan-Sonde F57 or LNA-Sonde F57
<300>
   <308> X70277
   <309> 1993-06-24
   <313> (1)..(620)
<400> 10
   ggatctcggc cccgatagct ttcctctcct tcgtcaccaa ctggcgcggg tccaggaacg    60
   cttggcactc gtcaatcacc acgaacacca gcggaacctg cggcgtcggg ccgcgatccc   120
   aaaagttgga cgatccgaat atgttgttgc cctgtctaat tcgatcacgg actagaccgg   180
   tcgcgtcatt cagaatcgct gcaatctcag gcagctccag atcgtcattc atgaatcggc   240
   agctacgagc acgcaggcat tccaagtcct gaccaccctt cccgtcgatg acagcgaact   300
   ggaccgccgc tgacgcaccg aacgacccca gagcactcgt cagccacgcg gttttccccg   360
   accctggtac gcctcccact acaacgcccg atacgttcgc caacgggagc gactggtaga   420
   cgcccatttc atcgataccc aaactcagag accacgagtg ggcggctggc acagacgacc   480
   attcaagcgg cgaactaagc ggatcgacaa ttctcagctg caactcgaac acacctggga   540
   ccggctcggt gacaacaaca ttcggtacac caatagctga cgccagcttc ggagccgcca   600
   cctgccactg tctgagatcc                                               620